# EUROPEAN PATENT SPECIFICATION

(11) **EP 1 305 130 B1**
(45) Date of publication and mention of the grant of the patent: **30.05.2007**
(21) Application number: 01909092.7
(22) Date of filing: 08.02.2001
(51) Int. Cl.: B23K 15/08, B23H 1/00, A61M 5/32

(54) **METHOD AND APPARATUS FOR DESTROYING NEEDLES**
VERFAHREN UND VORRICHTUNG ZUM ZERSTÖREN VON NADELN
PROCEDE ET APPAREIL DESTINES A LA DESTRUCTION D'AIGUILLES

(30) Priority: 09.02.2000 US 500504
(43) Date of publication of application: 02.05.2003
(73) Proprietor: Safeguard Medical Devices, Inc., Broadview Heights, OH 44147 (US)
(72) Inventor: ADKINS, Joey, B., Broadview Heights, OH 44147 (US)
(74) Representative: Freischem, Stephan
(86) International application number: PCT/US2001/004354
(87) International publication number: WO 2001/058637

(56) References cited:
- WO-A-96/33758
- WO-A1-97/33639
- FR-A- 2 770 407
- GB-A- 2 268 407
- GB-A- 2 297 230
- US-A- 5 076 178
- US-A- 5 138 125
- US-A- 5 212 362
- US-A- 5 336 862
- US-A- 5 736 706
- US-B1- 6 169 259

## Description

### BACKGROUND OF THE INVENTION

This invention generally relates to the destruction of biohazardous needles and, in particular, to the electrical destruction of used hypodermic needles. More specifically this invention relates to a device as defined in the preamble of claim 1 and to a method as defined in the preamble of claim 15.

Increasing emphasis has been placed on protecting patients and health professionals from needle sticks that may spread such pathogens as hepatitis and HIV. Various disposal containers have been used. Needles with various shielding schemes have been employed. Devices that cut needles have been tried.

To destroy the needle itself, devices have been used that melt the needle by connecting the needle across a large current source. Such devices require an extreme amount of current because of the relatively low resistance of the needle. This makes portable use impractical and line-powered power supplies expensive.

In addition, this destruction mechanism soon destroys the contacts of the device as well as the needles.

French Patent Publication No. 2 770 407, which corresponds to U.S. Patent No. 6,376,792, describes an apparatus 1 having a lower electrode 15 mounted in a fixed manner in an electrode-carrier cassette 3. The lower electrode 15 extends across a path for receiving a needle. A first upper electrode 18 is mounted in a fixed manner in the cassette 3. A second upper electrode 27 is mounted to a push button 4 for movement relative to the cassette 3. The second upper electrode 27 is connected to an electrode support 22 that rests an the lower electrode 15. A needle 12 contacts the lower electrode 15 and the first upper electrode 18 to melt the needle. Once an end piece 41 butts up against an upper face 44 of the first upper electrode 18, the push button 4 is actuated to move the second upper electrode 27 into contact with the needle 12. This Document describes electrodes 15, 18 and 27 in contact with a needle to melt the needle. According to Fig. 5b of the drawings of this document the needle remains open when its destruction is terminated so that residual liquid may spill from the interior of the syringe.

WO 96/33758 describes an apparatus having a first electrode 36 positioned coaxially under a hole 32 of a guide member 30. A second electrode 40 extends below the first electrode 36 and the hole 32. A needle is inserted through the hole 32 to bridge the gap between the electrodes 36 and 40 and short circuit the electrodes to melt the needle. Both electrodes 36 and 40 extend across a path for receiving the needle. This Document describes a method and device for destroying a needle by establishing a short circuiting with the needle. This consumes a rather large amount of energy. The electrodes 36 and 40 described in Document D2 are arranged adjacent to one another and to both contact the needle near the distal end.

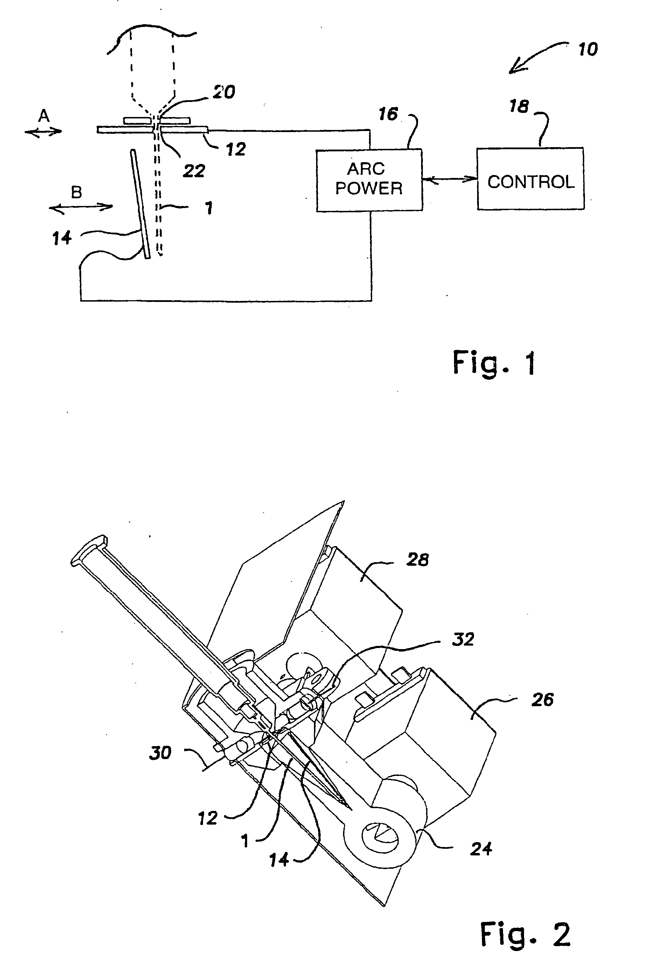

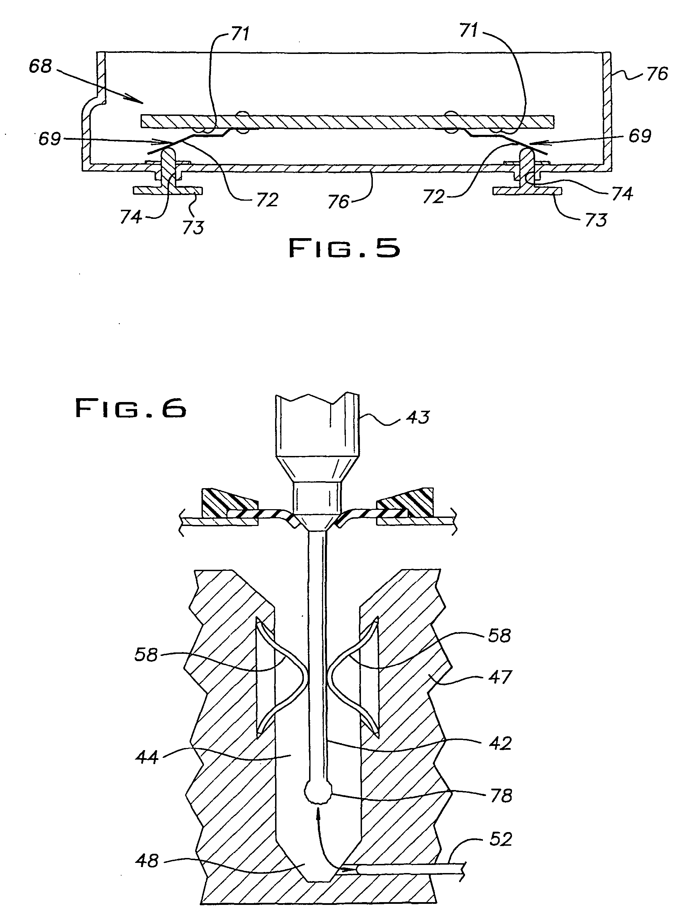

US. Patent No. 5,212,362 describes an apparatus having an enclosure 12 with an actuator 16 that receives a needle 18. The needle 18 is inserted into the actuator 16 and pressed down so that the actuator makes contact with a grounding bar 54. A solenoid 40 causes an electrode 28 to pivot toward the needle 18. The electrode 28 makes contact with the needle 18, which is grounded by contact with the actuator 16, to progressively volatize the end of the needle. The electrode 28 extends across a path for receiving the needle 18 along a longitudinal direction of the needle, see Fig. 2 of US 5,212,362. Accordingly, a needle may engage the electrode 28 and prevent the actuator 16 from being pressed down to make contact with the grounding bar 54. This document does not make any proposal for preventing discharge of any residual material that might exist in the remainder of the needle or piston chamber of a syringe. Due to the electrode movement the electrode 28 will always be in close contact with the needle until the needle has been volatilized so that the electrode can pass underneath said electrode (see Fig. 6). Fig. 6 does not show any obstruction of the bore of the needle so that residue liquid inside the syringe or the needle may escape.

### SUMMARY OF THE INVENTION

It is an object of the invention to overcome the disadvantages of the devices according to the prior art and in particular to provide a power efficient and durable device and method that reliably prevent discharge of any residual material that might exist in the remainder of the needle or piston chamber of a syringe.

This object is attained by the features of claim 1 with respect to the device and by the features of claim 15 with respect to the method. The sub-claims describe useful embodiments of the invention.

An apparatus for destroying a biohazardous needle having a proximal portion and a distal tip portion includes: an upper electrode adapted to contact the proximal portion; a lower electrode; and an electric arc supply connectable between the upper and lower electrodes. The supply is adapted to produce an electric arc between the lower electrode and the distal tip portion to destroy the needle.

A method for using said apparatus for destroying a biohazardous needle having a proximal portion and a distal tip portion includes: providing an electric arc supply having an upper and a lower output contact; connecting the upper output contact to the proximal portion; and creating an arc between the distal tip portion and the lower output contact where the arc progressively destroys the needle.

In a modified embodiment of the invention, the electrical arc treatment is essentially limited to the sharp distal end of a needle such as a hypodermic needle, that has been used and is, therefore, potentially biohazardous. In this method, the sharp end of the needle is first transformed into a molten mass globule which is then allowed to solidify almost instantly into an integral, blunt, closed cap on the remainder of the needle. The blunt character of the needle makes it "stick" resistant such that the risk that it will accidentally pierce a person's skin is greatly reduced.

Moreover, the fusion of the material into an integral cap prevents discharge of any residual material that might exist in the remainder of the needle or piston chamber of the syringe.

This point blunting embodiment simplifies the apparatus over that required to destroy the full length of a needle and to contend with the remnants of the needle material. The apparatus is simplified in large part because it need only receive a known length portion of any needle regardless of the needle's actual full length. With simple componentry and relatively small electrical power requirements to treat a needle tip, the invention can be embodied in a small portable device.

Such a device can be carried by a medical professional or assistant making it available where needles are used, ideally, right at a patient's side. This allows needles to be blunted immediately or shortly after use with relative ease. Consequently, the danger of accidental needle sticks is greatly minimized since the number of times a needle must be handled before it is blunted is reduced and the number of people potentially handling or otherwise exposed to a needle before it is blunted is reduced.

### BRIEF DESCRIPTION OF THE DRAWINGS

FIG. 1 is a schematic diagram of a needle destroying apparatus according to the invention;
FIG. 2 is a perspective view with portions cut away of an apparatus according to the invention;
FIG. 3 is a fragmentary cross-sectional view of a needle blunting apparatus according to a second embodiment of the invention receiving a sharp needle end;
FIG. 4 is a block diagram of the electrical power system of the second embodiment;
FIG. 5 is a cross-sectional view of a base of the apparatus of the second embodiment; and
FIG. 6 is a fragmentary cross-sectional view of the apparatus of the second embodiment, similar to FIG. 3, showing a blunted needle end.

### DESCRIPTION OF THE PREFERRED EMBODIMENTS

Referring to FIG. 1, a needle destroying apparatus 10 includes an upper electrode 12, a lower electrode 14, an arc power supply 16 and a control 18.

The needle 1 may be advantageously immobilized by a shutter effect between a stationary aperture 20 and an aperture 22 in the plate-like electrode 12 when the electrode 12 is moved along the direction A. The electrode 12 may be moved by various mechanisms, including, for example, a manually operated linkage or camming action or such other well-known techniques as an electrically-operated solenoid or motor and gear train or linkage. The upper electrode may be, for example, comprised of stainless steel. It would of course be possible to maintain the electrode 12 stationary and instead move the aperture 20 (i. e., a member containing the aperture 20)

The electrode 14 may advantageously be an elongated member lying generally in a plane through the needle 1.

The lower portion of the electrode 14 is located closer to the tip or distal portion of the needle 1 than is the upper portion of the electrode 14 with respect to the upper or proximal portion of the needle 1. In the preferred embodiment, the electrode 14 is a straight rod of a heat-resistant electrical conductor. The electrode may be, for example, stainless steel, or for higher heat resistance, tungsten. The electrode 14 may be, for example, a circular rod as long or longer than the needle to be destroyed and between 0.508 and 1.524 mm (0.020 and 0.060 inches) in diameter, with 0.8128 mm (0.032 inches) preferred.

The electrode 14 may be mounted on a carrier more fully described below. The electrode 14 may be moved along the direction B by various mechanisms, including, for example, a manually operated linkage or camming action or such other well-known techniques as an electrically-operated solenoid or motor and gear train or linkage. As more fully described below, movement of the electrode along the direction B facilitates the striking of an electric arc between the electrode 14 and the needle 1, as well as the removal of "ash" from the end of the needle 1.

The arc power supply 16 provides high voltage between the electrodes 12,14 sufficient to establish an air arc between the needle 1 and the electrode 14 while the proximal portion of the needle 1 is in electrical contact with the electrode 12. The arc supply 16 provides sufficient current in combination with the voltage to destroy the needle 1 using the heat of the arc to a point close to the electrode 12.

The arc supply 16 may include, for example, a battery or line powered oscillator driving a step-up transformer. The output of the arc supply may be, for example, about 25 milliamps at 800 volts for needles in the range of 27-32 gauge (0.406 - 0.229 mm outer diameter). Larger needles of 18-26 gauge (1.270-0.457 mm outer diameter) may require about 125 milliamps at 800 volts. In general, a maximum arc distance of about 6.35 mm (one-quarter inch) has been satisfactory. In the preferred embodiment, the arc supply 16 includes a full-wave rectifier that provides direct current to the electrodes 12,14 with the electrode 12 having a negative polarity with respect to the electrode 14.

The control 18 controls the operation of the apparatus 10. The control 18 may be, for example, simply a manual switch to control power to the arc supply 16 or it may be a more complex device as described more fully below.

In operation, the needle 1 of a syringe or other biohazardous needle is inserted into the aperture 20.

The electrode 12 is moved to contact the needle 1 trapping it between the walls of the aperture 20 and the aperture 22. The arc supply 18 is energized and the electrode 14 moved against the distal tip of the needle 1, temporarily shorting the arc supply 18, and the electrode 14 is then pulled away from the needle 1 thereby striking an arc between the needle 1 and the electrode 14. The resulting arc melts/burns the tip of the needle 1 and the arc continues up the portion of the electrode 14 closest to the remaining lower portion of the needle 1, progressively destroying the needle as the arc travels upward.

After the needle 1 is destroyed by the arc, the electrode 12 is released and the stub of the needle 1 withdrawn from the apertures 20,22.

The increasing distance between the electrode 14 and the needle 1 towards the upper proximal portion of the needle 1 helps ensure that the arc starts at the distal tip of the needle 1 and moves toward the proximal portion. This is due to a combination of air heated by the arc tending to push the arc upward balanced by the tendency for the arc to jump to the closest point between the electrode 14 and the needle 1. The needle/electrode are not required to be in a vertical plane but the proximal portion of the needle 1 should be higher than the distal tip. The angle between the needle 1 and the electrode 14 may be, for example, between 10 and 15 degrees with 12 degrees being satisfactory.

In the case of hollow hypodermic needles, the apparatus 10 has the further advantage that the progressive melting of the needle 1 results in a melted bead of metal that both blunts and seals any remaining portion of the needle 1.

By maintaining the electrode 12 negative with respect to the electrode 14, it has been found that the majority of the heat from the arc is transferred to the needle 1 instead of to the electrode 14, greatly improving the durability of the electrode 14.

It has been found that the use of an air arc to destroy the needle, as opposed to destroying it with a short circuit, requires much less power and greatly improves the durability of the contact electrodes. The lower power required makes it possible to operate the device for small gauge needles such as insulin syringes on a few AA batteries for hundreds of needles. In this low power configuration, it is desirable to move the electrodes 12,14 with a manually operated linkage that also operates a switch for the control 18.

Referring to FIG. 2 a solenoid-based embodiment of the invention includes a carriage 24 carrying the electrode 14 at the bottom of a v-shaped groove. The carriage 24 may be, for example, an electrically insulating plastic or, for higher heat resistance, a ceramic material. The carriage 24 is mounted on the plunger of a solenoid 26 and the electrode 12 is mounted to the plunger of a solenoid 28. A light emitting diode 30 and a phototransistor 32 are mounted about the needle 1.

In this case, the control 18 uses the diode 30 and the transistor 32 to detect the presence of a needle 1.

The control 18 energizes the solenoid 28 to move the electrode 12 to contact and grasp the needle 1. The control 18 also energizes the solenoid 26 to bring the electrode 14 into momentary contact with the needle 1 to strike the arc.

When operating with larger gauge needles, ash may be left in place of portions of the needle 1 resulting in interference with the progression of the arc. The solenoid 26 may also be energized by the control 18 to use the carriage 24 to periodically knock this ash loose.

For longer needle lengths, the electrode 14 can be advantageously increased in length also. This results in an electrode having an upper portion that would be much farther away from the needle 1 when the lower portion of the needle was in contact with or close to the distal tip of the long needle. This would then require a much higher voltage to sustain the arc at the proximal portion of the needle 1. This would in turn negatively impact the required power, the dissipated heat and the required electrical insulation and electronic component working voltages.

As an alternative, the electrode 14 can be initially positioned in an intermediate position suitable for shorter needles. Then a longer needle can be sensed by the control 18 when the distal tip of the needle contacts the electrode 14 prior to the electrode 14 being moved to strike an arc. The control 18 can then energize the solenoid 26 (or another unshown solenoid) to move the electrode 14 further away to accommodate the longer needle. If the arc to the proximal portion of the longer needle extinguishes because of the further distance, the control 18 can energize the solenoid 26 to move back to the intermediate position and to then strike a new arc.

Additional increments of movement by the electrode 14 can of course be employed to accommodate even a wider range of needle lengths.

The control 18 may include, for example, discrete logic or a microprocessor to perform the required control functions.

The present invention may be readily extended to neutralize not only biohazardous needles but also sharps in general such as sharp-edged surgical instruments like scalpels. Because of the large mass involved, the goal is to dull the cutting edge with an electrical arc rather than trying to destroy the whole blade.

In this case, rather than using an elongated electrode that roughly corresponds to the straight needle, a more localized (e. g., a point source) electrode is mechanically moved not just in one direction, but in two, to follow the contour of the cutting edge. Various edge tracking techniques can be used, but the preferred embodiment employs measuring the arc resistance to provide a measure of the distance between the electrode and the cutting edge. This measurement is then used to control servos that position the electrode. This device can of course also be used to destroy needles.

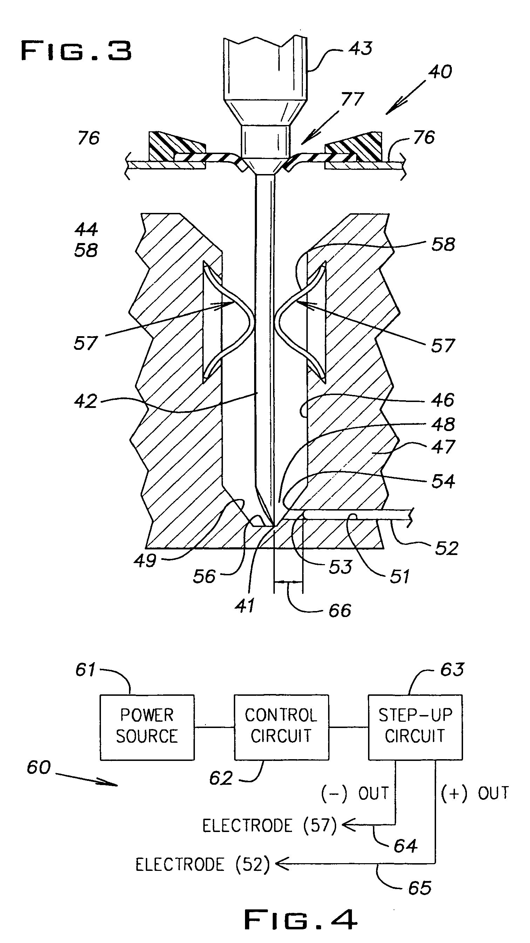

Referring now to FIG. 3, apparatus 40 for blunting a distal sharp end 41 of a hollow needle 42 of a hypodermic syringe 43 includes a path 44 for receiving the distal end and a portion of the needle rearward of the end. The path 44, in the illustrated arrangement, is generally vertical and is formed by generally vertical wall areas of a cylindrical bore 46 in a body 47. The body 47 is made of a rigid high temperature electrically insulating material such as ceramic material. The path 44 can take the illustrated form of the cylindrical walls of the bore 46 or can have other forms that, like the bore 46, are capable of guiding the lead or sharp end 41 of the needle 42 to a receiving zone 48. The illustrated receiving zone 48 is formed by a pocket in the body 47 with tapered or conical walls 49 that direct the needle tip or end 41 towards the center or axis of the path 44, which is coincident with the axis of the cylindrical bore 46, as the end 41 is lowered vertically into this zone.

A small bore 51 radial to the central axis of the path 44 is formed in the body 47 and receives an electrode 52. The electrode 52 is positioned in the bore 51 with its end 53 recessed slightly from an edge 54 where the bore 51 intersects with the wall 49 of the receiving zone 48. A terminal blade, screw or other suitable element (not shown) permits an electrical circuit discussed below to be connected to the electrode 52. A relatively flat bottom 56 of the receiving zone 48 arrests axial or longitudinal motion of the needle 42 into the receiving zone.

Associated with the path 44 is an electrode 1 comprising a pair of opposed electrical conductors 58 in the form of flat blade metal springs. The electrode springs 58 have arcuate configurations that are normally configured to contact or nearly contact one another from opposite sides of the path 44. As shown, the electrode springs 57 in both axial directions along the path 44 extend away from the longitudinal center of the path 44 formed by the axis of the cylindrical bore 46. The electrode springs 58 are each provided with a terminal blade or other suitable element (not shown) for connecting it to the electrical circuit discussed below.

FIG. 4 illustrates an electrical power system 60 for developing a destructive needle consuming arc. The power system 60 includes a power source 61, a control circuit 62 and a voltage step-up circuit 63. The power source 61, in the preferred embodiment, comprises a DC battery which can be of a conventional dry cell type or a conventional rechargeable type. The control circuit 62 in its simplest form can be a manually operated switch.

The step-up circuit 63, of any suitable type of known electrical circuits, increases the voltage supplied by the power source 61 through the control circuit 62. The voltage can be increased to about 2,000 volts, open circuit, by way of example, where a gap 66 is initially about 1.5875 mm (1/16 inch) with the step-up circuit 63. Depending on the size of the gap 66, other voltages between about 1,000 and 10,000 volts, and even as high as 40,000 volts, open circuit, can be employed. The voltage output of the step-up circuit 63 is connected to the electrodes 52,57 by high voltage lines 64,65 with the negative side preferably applied to the spring blade electrode elements 58.

FIG. 5 illustrates an optional part 68 of the control circuit 62 that can be used to reduce the risk that the device or apparatus 40 will be used while being "hand-held" and thereby reduce the risk of accidental needle sticks. This control circuit part 68 includes a set of four electrical switches 69 (two are shown in the plane of FIG. 5) that sense whether or not the apparatus 40 is supported on a flat surface. The switches include fixed contacts 71 and movable spring blade contacts 72.

The switches 69 are normally open by virtue of the configuration of the blade 72. Associated with each switch 69 is a foot or button 73 vertically movable in a hole 74 in a housing or base 76 in which other parts of the apparatus 40 are contained. The switches 69 are connected in series with each other and with the electrical power source 61. Therefore, unless all four feet 73 are positively moved into the housing 76 to close the switches 69, from the illustrated position in FIG. 5, there is no electrical power delivered to the step-up circuit 63. The weight of the apparatus 40 is sufficient to deflect all of the spring contact blades 72 to close the switches 69 when the apparatus 40 is placed on a flat surface. However, if the apparatus is held directly in a person's hand, all of the feet 73 will not be readily supported from underneath so that the apparatus 40 will not ordinarily operate in such a situation. This feature discourages the practice of using the device while holding it in a person's hand.

In operation, a needle is inserted through an opening 77 in the housing 76 and is moved vertically downwardly and longitudinally along the path 44. The sharp distal end 41 of the needle 42 moves through a space between the opposed electrode blades 57 until this end rests against the flat bottom 56 of the receiving zone 48. The control circuit 62 is energized, such as by operation of a manual switch, to apply voltage from the step-up circuit 63 across the electrodes 52,57. The spring character of the electrode blades 58 establishes and maintains electrical contact with the exterior surface of the needle which is typically an electrically conductive metal. The voltage applied across the electrodes 52,57 is preferably high enough to enable an arc to jump across the gap between the electrode 52 and the needle tip or end 41. The arc is maintained for a time and power level sufficient to melt the sharp point of the end 41 into a molten mass. The arc extinguishes and the mass agglomerates into a somewhat spherically shaped solid 78 which is thus blunt and serves to weld or cap the hollow needle closed. The result of this action is shown in FIG. 6. By way of example, an arc power of approximately 40 watts can be used to achieve sufficient blunting of a needle 42, requiring about 1/2 second for a typical 27 gauge (0.406 mm outer diameter) needle to about 1-1/2 seconds for a typical 18 gauge (1.270 mm outer diameter) needle. Particularly where the needle 42 is relatively small, the end of the needle may be melted or otherwise consumed by the arc such that the needle recedes away from the electrode 52 up the length of the needle to a point where the length of the arc by a line of sight from this electrode is greater than that which the voltage can sustain, at which point the arc extinguishes itself. The curved configuration of the blades 58 enable the needle end to be withdrawn regardless of a moderate enlargement of its diameter at the fused solid 78 where it is blunted. Other configurations of the electrode 57 are contemplated.

These include a single blade, or a metal roll or rollers.

The voltage supplied by the step-up circuit 63 can be regulated so that an initial high voltage can be used to establish an arc and then the current or power delivered can be reduced to obtain the desired results.

The disclosed device is somewhat self-regulating because as the agglomerated mass at the lower end of the needle 42 increases in size, the energy it dissipates by radiation and convection approaches the energy coming from the arc. If the mass becomes large enough to reach the wall of the receiving zone 48 or cylindrical bore 46, the material will solidify almost instantly through conduction and melting of the needle will cease. Where a needle is relatively large size, limited feeding of the needle towards the electrode 52 can be performed to ensure that a solid sealed cap is produced. If desired, physical contact between the needle tip and the electrode can be made by a suitable mechanism to strike an initial arc so that a relatively high voltage is not required to start the arc.

While the invention has been shown and described with respect to particular embodiments thereof, this is for the purpose of illustration rather than limitation, and other variations and modifications of the specific embodiments herein shown and described will be apparent to those skilled in the art all within the scope of the invention as defined in the claims.

## Claims

1. A device for blunting a hypodermic needle (1,42) comprising a base (76), a path (44) on the base (76) for receiving the distal end (41) of the needle (1,42) along a longitudinal direction of the needle (1,42), a first electrode (12,57) adapted to engage the needle (1,42) when the latter is inserted into the path (44), a second electrode (14,52) adjacent the path (44) of the needle (1,42), a supply of electrical energy, the supply being electrically connected to the electrodes (12,57;14,52) and being arranged to apply a voltage across the electrodes (12,57;14,52), **characterized in that** said second electrode (14,52) is spaced from the path (44) of the needle (1,42) with a gap between said second electrode (14,52) and said needle (1,42) and that the power supply, needle receiving path (44), and electrode positioning being arranged to establish a destructive air arc across the gap that melts and agglomerates the distal end of the needle (1,42) to render it blunt and effectively block its bore.

2. A device according to claim 1, wherein said arc seals said needle (1,42).

3. A device according to claim 1 or 2, wherein said electric arc supply is adapted to provide said upper electrode (12,57) with a negative polarity with respect to said lower electrode (14,52).

4. A device according to any one of the preceding claims, further comprising a second electrode carriage (24), said carriage (24) being adapted to move said second electrode into momentary contact with said needle (1) to strike said air arc.

5. A device according to claim 4, wherein said carriage (24) is further adapted to move said second electrode (14) toward said needle (1) to remove ash therefrom.

6. A device according to claim 4, wherein said carriage (24) is further adapted to move said second electrode (12) a further distance from said needle (1) if said needle (1) contacts said second electrode (12) before said second electrode (12) is moved into momentary contact with said needle (1).

7. A device according to any one of the preceding claims, wherein said second electrode (12) includes an elongate member having a lower portion and an upper portion, said elongate member being adapted to maintain said lower portion closer to said distal tip portion than said upper portion to progressively destroy said needle (1).

8. A device as set forth in any one of the preceding claims, including an electrically insulating material (47) that shields the second electrode (52) from direct contact with the sharp distal end (41) of the needle (42).

9. A device as set forth in any one of the preceding claims, wherein said path (44) has a longitudinal direction, said second electrode (52) being laterally displaced from said path (44).

10. A device as set forth in any one of the preceding claims, including sensing elements (68) for verifying that the device is supported on a flat surface.

11. A device as set forth in claim 10, wherein said sensing elements (68) are arranged to inhibit actuation of said arc when said sensing elements (68) indicate that said device is not resting on a flat surface.

12. A device as set forth in any one of the preceding claims, wherein said first electrode (57) is resiliently biased by spring action into said path.

13. A device as set forth in claim 12, wherein said first electrode comprises a pair of opposing electrically conductive elements (57).

14. A device as set forth in claim 13, wherein said first electrode (57) has surfaces (58) that diverge laterally from a longitudinal direction of the path (44) such that the tip (41) of a needle (42) causes the surfaces (58) to deflect laterally when the tip (41) passes in or out of the path (44).

15. A method of blunting a biohazardous hypodermic needle (1,42) or other sharp instrument comprising the steps of contacting the surface of the needle (1,42) at a location anterior of its sharp end (41) with a first electrode (12,57), disposing the sharp end (41) of the needle (1,42) adjacent a second electrode (14,52), **characterized in that** said second electrode (14,52) is spaced from the path (44) of the needle (1,42) with a gap between said second electrode (14,52) and said needle (1,42) and that an air arc is established between the second electrode (14,52) and the sharp tip (41) of the needle (1,42) by a voltage across the electrodes (12,57;14,52) to melt the sharp tip (41) of the needle (42) into an agglomerated mass to render it blunt and effectively block its bore.

16. A method as set forth in claim 15, wherein the first electrode (12,57) is arranged at a negative potential relative to the second electrode (14,52).

17. A method as set forth in claim 15 or 16, wherein the needle (1,42) is moved longitudinally along a path (44) to where the sharp tip (41) is caused to enter a receiving zone and the second electrode (52) is spaced laterally of said path (44).

## Patentansprüche

1. Vorrichtung zum Abstumpfen einer hypodermischen Nadel (1,42), umfassend eine Basis (76), eine Bahn (44) an der Basis (76) zur Aufnahme des distalen Endes (41) der Nadel (1,42) entlang einer Längsrichtung der Nadel (1,42), eine erste Elektrode (12,57), welche dazu angepaßt ist, mit der Nadel (1,42) in Eingriff zu kommen, wenn letzere in die Bahn (44) eingeführt wird, eine zweite Elektrode (14, 52) nahe der Bahn (44) der Nadel (1, 42) und eine elektrische Stromversorgung, wobei die Stromversorgung mit den Elektroden (12, 57; 14, 52) elektrisch verbunden ist und angeordnet ist, um eine Spannung an die Elektroden (12, 57; 14, 52) anzulegen, **dadurch gekennzeichnet, daß** die genannte zweite Elektrode (14, 52) in einem Abstand zur Bahn (44) der Nadel (1, 42), mit einem Spalt zwischen der genannten zweiten Elektrode (14, 52) und der genannten Nadel (1, 42), angeordnet ist und daß die Stromversorgung, die Bahn zur Aufnahme der Nadel (44) und die Positionierung der Elektroden so angeordnet sind, daß sie quer über den Spalt einen zerstörenden Lichtbogen erzeugen, welcher das distale Ende der Nadel (1, 42) schmilzt und zusammenklumpt, um es stumpf zu machen und ihre Bohrung effektiv zu verschließen.

2. Vorrichtung nach Anspruch 1, wobei der genannte Lichtbogen die genannte Nadel (1, 42) abdichtet.

3. Vorrichtung nach Anspruch 1 oder 2, wobei die genannte Stromversorgung für den Lichtbogen angepaßt ist, um die genannte obere Elektrode (12, 57) mit einer negativen Polarität in bezug auf die genannte untere Elektrode (14, 52) zu versorgen.

4. Vorrichtung nach einem der vorangehenden Ansprüche, welche ferner eine Halterung für die zweite Elektrode (24) aufweist, wobei die genannte Halterung (24) angepaßt ist, die genannte zweite Elektrode so zu bewegen, daß sie kurzzeitig in Kontakt mit der genannten Nadel (1) kommt, um den genannten Lichtbogen zu zünden.

5. Vorrichtung nach Anspruch 4, wobei die genannte Halterung (24) ferner dazu angepaßt ist, die genannte zweite Elektrode (14) auf die genannte Nadel (1) zuzubewegen, um Asche von ihr zu entfernen.

6. Vorrichtung nach Anspruch 4, wobei die genannte Halterung (24) ferner dazu angepaßt ist, die genannte zweite Elektrode (12) weiter von der genannten Nadel (1) zu entfernen, wenn die genannte Nadel (1) in Kontakt mit der genannten zweiten Elektrode (12) kommt bevor die genannte zweite Elektrode (12) bewegt wird, um kurzzeitig mit der genannten Nadel (1) in Kontakt zu kommen.

7. Vorrichtung nach einem der vorangehenden Ansprüche, wobei die genannte zweite Elektrode (12) ein längliches Element mit einem unteren Abschnitt und einem oberen Abschnitt aufweist, wobei das genannte längliche Element angepaßt ist, den genannten unteren Abschnitt näher als den genannten oberen Abschnitt an dem genannten distalen Spitzenabschnitt zu halten, um die genannte Nadel (1) nach und nach zu zerstören.

8. Vorrichtung nach einem der vorangehenden Ansprüche, die ein elektrisch isolierendes Material (47) aufweist, daß die zweite Elektrode (52) von direktem Kontakt mit dem spitzen distalen Ende (41) der Nadel (42) abschirmt.

9. Vorrichtung nach einem der vorangehenden Ansprüche, wobei die genannte Bahn (44) eine Längsrichtung aufweist und die genannte zweite Elektrode (52) seitlich versetzt zu der genannten Bahn (44) angeordnet ist.

10. Vorrichtung nach einem der vorangehenden Ansprüche, welche Abtastelemente (68) aufweist, um zu überprüfen, ob die Vorrichtung auf einer ebenen Oberfläche gelagert ist.

11. Vorrichtung nach Anspruch 10, wobei die genannten Abtastelemente (68) so angeordnet sind, daß sie die Zündung des genannten Lichtbogens verhindern wenn die genannten Abtastelemente (68) anzeigen, daß die genannte Vorrichtung nicht auf einer ebenen Oberfläche lagert.

12. Vorrichtung nach einem der vorangehenden Ansprüche, wobei die genannte erste Elektrode (57) elastisch federnd in der genannten Bahn vorgespannt ist.

13. Vorrichtung nach Anspruch 12, wobei die genannte erste Elektrode ein Paar gegenüber angeordnete elektrisch leitende Elemente (57) aufweist.

14. Vorrichtung nach Anspruch 13, wobei die genannte erste Elektrode (57) Oberflächen aufweist, die seitlich von der Längsrichtung der Bahn (44) abweichen, so daß die Spitze (41) einer Nadel (42) bewirkt, daß die Oberflächen (58) seitlich ausweichen, wenn die Spitze (41) in die Bahn (44) eingeführt oder aus der Bahn (44) herausgezogen wird.

15. Verfahren zum Abstumpfen einer biogefährlichen hypodermischen Nadel (1,42) oder eines anderen spitzen Instruments, welches die Schritte umfaßt, die Oberfläche der Nadel (1,42) an einer Stelle vor ihrem spitzen Ende (41) mit einer ersten Elektrode (12, 57) zu kontaktieren, das spitze Ende (41) der Nadel (1, 42) nahe einer zweiten Elektrode (14, 52) anzuordnen, **dadurch gekennzeichnet, daß** die genannte zweite Elektrode (14, 52) in einem Abstand zur Bahn (44) der Nadel (1, 42), mit einem Spalt zwischen der genannten zweiten Elektrode (14, 52) und der genannten Nadel (1, 42), angeordnet ist und daß durch eine Spannung zwischen den Elektroden (12, 57; 14, 52) ein Lichtbogen zwischen der zweiten Elektrode (14, 52) und der spitzen Spitze (41) der Nadel (1, 42) erzeugt wird, um die spitze Spitze (41) der Nadel (42) zu einer zusammengeklumpten Masse zu schmelzen mit dem Ziel, sie stumpf zu machen und ihre Bohrung effektiv zuverschließen.

16. Verfahren nach Anspruch 15, wobei die erste Elektrode (12, 57) auf ein negatives Potential in bezug auf die zweite Elektrode (14, 52) gebracht ist.

17. Verfahren nach Anspruch 15 oder 16, wobei die Nadel (1, 42) längs entlang einer Bahn (44) dorthin bewegt wird, wo die spitze Spitze (41) in eine Aufnahmezone eintritt und die zweite Elektrode (52) seitlich in einem Abstand zur genannten Bahn (44) angeordnet ist.

## Revendications

1. Dispositif pour émousser une aiguille hypodermique (1, 42) comportant une base (76), un trajet (44) sur la base (76) pour recevoir l'extrémité distale (41) de l'aiguille (1, 42) le long d'une direction longitudinale de l'aiguille (1, 42), une première électrode (12, 57) adaptée pour venir en prise avec l'aiguille (1, 42) lorsque cette dernière est insérée dans le trajet (44), une seconde électrode (14, 52) adjacente au trajet (44) de l'aiguille (1, 42), une alimentation d'énergie électrique, l'alimentation étant reliée électriquement aux électrodes (12, 57 ; 14, 52) et étant agencée pour appliquer une tension à travers les électrodes (12, 57 ; 14, 52), **caractérisé en ce que** ladite seconde électrode (14, 52) est espacée du trajet (44) de l'aiguille (1, 42) par un espace situé entre ladite seconde électrode (14, 52) et ladite aiguille (1, 42), et **en ce que** l'alimentation électrique, le trajet de réception d'aiguille (44), et le positionnement des électrodes sont prévus pour établir un arc à l'air libre destructif à travers l'espace qui fait fondre et agglomère l'extrémité distale de l'aiguille (1, 42) afin de la rendre émoussée, et de bloquer effectivement son alésage.

2. Dispositif selon la revendication 1, dans lequel ledit arc scelle ladite aiguille (1, 42).

3. Dispositif selon la revendication 1 ou 2, dans lequel ladite alimentation d'arc électrique est adaptée pour doter ladite électrode supérieure (12, 57) d'une polarité négative par rapport à ladite électrode inférieure (14, 52).

4. Dispositif selon l'une quelconque des revendications précédentes, comportant en outre un second chariot d'électrode (24), ledit chariot (24) étant adapté pour déplacer ladite seconde électrode en contact momentané avec ladite aiguille (1) afin d'heurter ledit arc à l'air libre.

5. Dispositif selon la revendication 4, dans lequel ledit chariot (24) est en outre adapté pour déplacer ladite seconde électrode (14) vers ladite aiguille (1) afin d'enlever de la cendre à partir de celle-ci.

6. Dispositif selon la revendication 4, dans lequel ledit chariot (24) est en outre adapté pour déplacer ladite seconde électrode (12) à une distance supplémentaire de ladite aiguille (1) si ladite aiguille (1) vient en contact avec ladite seconde électrode (12) avant que ladite seconde électrode (12) ne soit déplacée en contact momentané avec ladite aiguille (1).

7. Dispositif selon l'une quelconque des revendications précédentes, dans lequel ladite seconde électrode (12) comporte un élément allongé ayant une partie inférieure et une partie supérieure, ledit élément allongé étant adapté pour maintenir ladite partie inférieure plus proche de ladite partie de pointe distale que ladite partie supérieure, pour détruire progressivement ladite aiguille (1).

8. Dispositif selon l'une quelconque des revendications précédentes, comportant un matériau électriquement isolant (47) qui protège la seconde électrode (52) d'un contact direct avec l'extrémité distale aiguë (41) de l'aiguille (42).

9. Dispositif selon l'une quelconque des revendications précédentes, dans lequel ledit trajet (44) a une direction longitudinale, ladite seconde électrode (52) étant déplacée latéralement par rapport audit trajet (44).

10. Dispositif selon l'une quelconque des revendications précédentes, comportant des éléments de détection (68) pour vérifier que le dispositif est supporté sur une surface plate.

11. Dispositif selon la revendication 10, dans lequel lesdits éléments de détection (68) sont agencés pour empêcher un actionnement dudit arc lorsque lesdits éléments de détection (68) indiquent que ledit dispositif ne repose pas sur une surface plate.

12. Dispositif selon l'une quelconque des revendications précédentes, dans lequel ladite première électrode (57) est rappelée de manière élastique par une action de ressort dans ledit traj et.

13. Dispositif selon la revendication 12, dans lequel ladite première électrode comporte une paire d'éléments électriquement conducteurs opposés (57).

14. Dispositif selon la revendication 13, dans lequel ladite première électrode (57) a des surfaces (58) qui divergent latéralement d'une direction longitudinale du trajet (44), de sorte que la pointe (41) d'une aiguille (42) amène les surfaces (58) à se déformer latéralement lorsque la pointe (41) passe dans le trajet (44), ou à l'extérieur de celui-ci.

15. Procédé pour émousser une aiguille hypodermique dangereuse pour l'organisme (1, 42) ou un autre instrument aigu, comportant les étapes consistant à mettre la surface de l'aiguille (1, 42), au niveau d'un emplacement antérieur de son extrémité aiguë (41), en contact avec une première électrode (12, 57), à disposer l'extrémité aiguë (41) de l'aiguille (1, 42) adjacente à une seconde électrode (14, 52), **caractérisé en ce que** la seconde électrode (14, 52) est espacée du trajet (44) de l'aiguille (1, 42) par un espace situé entre ladite seconde électrode (14, 52) et ladite aiguille (1, 42), et **en ce qu'**un arc à l'air libre est établi entre la seconde électrode (14, 52) et la pointe aiguë (41) de l'aiguille (1, 42) par une tension à travers les électrodes (12, 57 ; 14, 52) pour faire fondre la pointe aiguë (41) de l'aiguille (42) en une masse agglomérée afin de la rendre émoussée, et de bloquer effectivement son alésage.

16. Procédé selon la revendication 15, dans lequel la première électrode (12, 57) est agencée à un potentiel négatif par rapport à la seconde électrode (14.52).

17. Procédé selon la revendication 15 ou 16, dans lequel l'aiguille (1, 42) est déplacée longitudinalement le long d'un trajet (44) par lequel la pointe aigüe (41) est amenée à entrer dans une zone de réception, et la seconde électrode (52) est espacée latéralement dudit trajet (44).
